Europäisches Patentamt

(19) European Patent Office

Office européen des brevets

(11) Publication number: **0 133 671 B1**

# EUROPEAN PATENT SPECIFICATION

(45) Date of publication of patent specification: 24.07.91  (51) Int. Cl.⁵: **C12Q 1/68**

(21) Application number: **84108890.9**

(22) Date of filing: **27.07.84**

The file contains technical information submitted
after the application was filed and not included in
this specification

(54) Detection of bacteria by nucleic acid hybridization, labeled probes and test kit therefore.

(30) Priority: **05.08.83 US 520524**

(43) Date of publication of application:
**06.03.85 Bulletin  85/10**

(45) Publication of the grant of the patent:
**24.07.91 Bulletin  91/30**

(84) Designated Contracting States:
**CH DE FR GB IT LI NL SE**

(56) References cited:
**EP-A- 0 076 123**
**WO-A-83/01459**

**THE JOURNAL OF BIOLOGICAL CHEMISTRY,
vol. 255, no. 2, 25th January 1980, pages
728-734, USA; D. FILER et al.: "Portions of the
gene encoding elongation factor Tu are
highly conserved in prokaryotes"**

**PROCEEDINGS OF THE NATIONAL ACADEMY
OF SCIENCES OF THE USA, vol. 78, no. 6,
June 1981, pages 3751-3754; W.D. STUART et
al.: "Location of the 18/28S ribosomal RNA
genes in two Hawaiian Drosophila species
by monoclonal immunological identification
of RNA-DNA hybrids in situ"**

**ADVANCES IN GENETICS, vol. 16, 1972,
pages 81-118; D.J. BRENNER et al.:
"Molecular relationships among members of
the enterobacteriaceae"**

(73) Proprietor: **MILES INC.**
**1127 Myrtle Street**
**Elkhart Indiana 46514(US)**

(72) Inventor: **Carrico, Robert J.**
**54256 Silver Street**
**Elkhart, IN 46514(US)**
Inventor: **DeRiemer, Leslie H.**
**9623 Summit Circle**
**La Mesa, CA 92041(US)**
Inventor: **Grosch, Josephine C.**
**57113 Rt. 7-DeCamp Blvd.**
**Elkhart, IN 46514(US)**
Inventor: **Wilson, Gary A.**
**54401 Susquehanna Road**
**Elkhart, IN 46514(US)**

(74) Representative: **Adrian, Albert, Dr. et al**
**c/o BAYER AG Konzernverwaltung RP Paten-
tabteilung**
**W-5090 Leverkusen 1, Bayerwerk(DE)**

**Description**

BACKGROUND OF THE INVENTION

*1. FIELD OF THE INVENTION*

This invention concerns analytical methods and test means for detecting the presence of bacteria in a test sample, particularly, in areas related to health care, such as in the analysis of human body fluids, e.g., urine and blood, for the purpose of aiding diagnosis, or in the testing of foods to detect contamination.

*2. DESCRIPTION OF THE PRIOR ART*

The classical method for detecting bacteria in a test sample involves culturing of the sample in order to expand the number of bacterial cells present into observable colony growths which can be enumerated. If desired, the cultures can also be subjected to additional testing in order to determine antimicrobial susceptibility and identification of particular bacterial species. Bacteriological culture methods are particularly labor intensive and require highly skilled technicians. Normally, 24 to 48 hours of incubation are required before a positive or negative result can be reliably determined. Other limitations of the culture method include the need to maintain viability of bacteria in the test sample during transport to and processing at the laboratory.

A wide variety of alternative techniques have been investigated and developed in the continuing attempts to devise methods which overcome the numerous drawbacks of the conventional culture approach. Light microscopy is frequently used to detect bacteria in clinical specimens. Usually the specimen is stained to improve the detection limit and help characterize the organisms present. The method is laborious and does not offer good detection limits. Immunological methods have been successfully developed to detect specific species and genera which have surface antigens which are distinguishable by specific antibody binding. Such an approach is not practical, however, for quantitating bacteria in a test sample which may contain bacteria from a variety of genera which do not share a common surface antigen from such sources as in body fluids, e.g., blood or urine.

Other tests have been developed based on detection of metabolic products of bacteria such as nitrites, nucleotides such as adenosine triphosphate, $^{14}CO_2$ released from $^{14}C$-labeled glucose, and specific enzymes. The disadvantages of these techniques are many and include: ATP can be degraded by enzymes in the test sample, ATP can originate from nonbacterial sources such as tissues of the host, radioactive materials present a biohazard, and enzymes with similar activity can arise from the host.

Particle counting instrumentation has also been applied to detection of bacteria. Such instrumentation measures perturbations in an electrical current across a small orifice caused by the presence of particles in a fluid flowing through the orifice. Besides requiring the use of complex and expensive apparatus, this method is highly nonspecific and requires a high level of care and particle-free conditions.

Other types of instrumentation measure the growth of bacteria in special liquid media. The instruments involved make periodic turbidimetric measurements with increases in turbidity indicating the presence of growing bacteria.

Therefore, there is a continuing, long-felt need for a rapid, accurate, and economical technique and means for quantitating bacterial presence. Despite the rapid advance of analytical techniques in closely related fields, as represented by the developments of the radioimmunoassay, spectrophotometry, fluorometry, microcalorimetry, and electrochemical techniques, the predominant technique used in bacteriological testing remains the plate culture method, which today is still much the same as the techniques used by Pasteur and the other early microbiologists of the 19th century. A recent analysis of the state-of-the-art in microbiology concluded that the needs of modern medicine could best be served by developing alternatives to culturing for detecting microorganisms [*Nature 302*(1983) p. XXVII].

In 1964, Nygaard and Hall [*J. Mol. Biol. 9*:125-142 (1964)] described a method for quantitative detection of DNA/RNA hybrids by filtration of samples through nitrocellulose membranes. They discovered that single-stranded DNA adsorbs onto the membrane, whereas double-stranded (duplexed) DNA and single- or double-stranded RNAs pass through. Sample DNA in single-stranded form and radiolabeled RNA were allowed to incubate in solution resulting in hybridization binding between the radiolabeled RNA and complementary DNA strands in the sample. The reaction mixture was then passed through the nitrocellulose filter. The unhybridized labeled RNA passed through while the DNA-RNA hybrids had sufficient single-stranded DNA regions to adhere to the filter. Measurement of radioactivity bound to the filter provided an estimate of the amount of hybrid formed.

The birth of this nucleic acid hybridization technique was followed by further refinements and applications to specific analytical situations. Gillespie and Spiegelman [*J. Mol. Biol. 12*:829-843(1965)] fixed single-stranded sample DNA onto nitrocellulose filters and then submerged the filters into a solution of radio-labeled RNA to allow hybridization to occur. Excess labeled RNA was removed by treatment with RNase and washing. A DNA/DNA hybridization method on membrane filters was developed by Denhardt [*Biochem. Biophys. Res. Commun. 23*:641-646 (1966)]. Single-stranded sample DNA was bound to nitrocellulose and treated with a prehybridization solution to minimize nonspecific binding of labeled probe DNA during the hybridization reaction. Grunstein and Hogness [*Proc. Natl. Acad. Sci. USA 72*:3961-3965-(1975)] developed a solid phase colony hybridization technique for rapid screening of *E. coli* colonies to determine which ones contained an inserted DNA sequence. Immobilization of nucleic acids to solid supports has also been improved to include RNA and short DNA fragments. The techniques developed have included using paper with chemically reactive groups [Alwine *et al, Proc. Natl. Acad. Sci. USA 74*:4350(1977), Seed, *Nucleic Acids Res. 10*:1979(1982), and Hunger, *Bioch. Biophys. Acta 653*:344-(1981)] and derivatization of the nucleic acid prior to fixation on nitrocellulose [*Thomas, Proc. Natl. Acad, Sci. USA 77*:5201(1980)].

Nucleic acid hybridization assays have been developed for a number of diseases. These include the genetic diseases α-thalassemia [Kan *et al, New Eng. J. Med. 295*:1165(1976)], β-thalassemias [Orkin *et al, Nature 296*:627(1982)] and sickle cell anemia [Kan and Dozy, *Proc. Natl. Acad. Sci. USA 75*:5631 (1978), Geever *et al, ibid 78*:5081(1981), Wilson *et al, ibid 79*:3628(1982) and Conner *et al, ibid 80*:278 (1983)]. Nucleic acid hybridization tests for the presence of infectious agents have also been reported. These include a test for enterotoxin-producing bacteria in fecal specimens [Moseley *et al, J. Infect. Dis. 42*:892-(1980), *ibid 145*:563(1982), and U.S. Pat. No. 4,358,535]. See also Institut Pasteur, British Pat. 2,019,408B. Hepatitis virus in serum has also been detected by hybridization [Schafritz *et al, Proc. Natl. Acad. Sci. USA 79*:5675(1982) and Beminger *et al, J. Med. Virol. 9*:57(1982)].

Some studies have been undertaken of the generic relatedness of various bacteria based on nucleic acid hybridization between denatured and fragmented single-stranded DNA from different bacteria [see Brenner, *The Public Health Laboratory,* Thompson Publ. Inc., vol. 32 (1974) pp. 118-130; and the review by Brenner and Falkow, *Adv. in Genetics 16*:81-119(1972)]. Conservation of bacterial genomic DNA coding for various products such as ribosomal RNA, ribosomal proteins, transfer RNAs, and the like has been the subject of some study [*Biophys. J. 8*:1105-1118(1968); PNAS 76: 381-385(1979); *J. Bact. 133*:1089(1978); *J. Biol. Chem. 255*:728-734(1980); and *J. Bact. 129*:1435(1977)]. Filer *et al [Eur. J. Biochem. 120*:79-77-(1981)] have reported that DNA sequences homologous to certain prokaryotic genes, e.g., *E. coli tuf* genes, are present in some taxonomically unrelated genera.

## SUMMARY OF THE INVENTION

It has now been found that nucleic acid hybridization techniques can be applied to the task of quantifying bacteria in a variety of test samples, particularly in media of diagnostic significance such as body fluids. The present method therefore provides a highly convenient screening test for determining if there is significant bacterial presence in a test medium of interest, partidularly media which are normally sterile such as blood or urine.

The test sample is first subjected to conditions to release and denature (render single stranded) RNA from bacteria present in the sample. The resulting single stranded RNA is then contacted in an appropriate manner with a DNA probe having a base sequence which is homologous with at least one base sequence in the RNA content of substantially all of the various respective bacteria possibly present in the test sample. Contact between the probe and the denatured sample RNA is performed under conditions favorable to hybridization between the probe and the aforesaid respective bacterial base sequences. Resulting hybridization is then determined as a function of the extent of bacterial presence in the test sample.

The homologous base sequence of the probe is preferably at least about 10, and normally at least about 20, bases in length. The DNA probe can be selected in several manners including empirical screening of the bacteria expected to be present in the test sample for a homologous sequence or sequences or predetermined selection of a conserved or substantially conserved gene.

It has most particularly been found that a most advantageous nucleic acid hybridization assay for detecting bacteria is provided by the use of a probe comprising at least a portion of one of the strands of a gene which codes for the synthesis of a nucleic acid or protein comprised in the mechanism of protein synthesis. Important examples of such genes include those coding for transfer RNAs, ribosomal RNAs, ribosomal proteins, aminoacyl-tRNA synthetases, initiation factors, elongation factors, and releasing factors.

The present method for detecting bacteria has numerous advantages over the prior art techniques, particularly the classical culturing methods. In the present method it is not necessary to isolate or propagate the bacteria to be detected, which in some cases can be a highly infectious agent. Furthermore, it is not necessary to rely on the expression of certain metabolic products or surface antigens in order to make a determination. In addition, the present method does not require that test samples be handled in a manner necessary to guarantee viability of any bacteria present. The prior art methods in general will detect only viable cells in the sample. Often, microbes die during transport of specimens to the laboratory for testing. Also, a specimen can contain different types of microorganisms having a variety of growth limitations and requirements. Thus, when using the prior art methods, the specimen must be cultured under several different conditions in order to ensure detection of any possible bacteria present. Such a serious limitation is absolutely not required by the present method since the object of the assay is the stable nucleic acid composition of the bacteria present in a sample.

## DESCRIPTION OF THE PREFERRED EMBODIMENTS

The use of nucleic acid hybridization as an analytical tool is based fundamentally on the double-stranded, duplex structure of DNA. The hydrogen bonds between the purine and pyrimidine bases of the respective strands in double-stranded DNA can be reversibly broken. The two complementary single strands of DNA resulting from this "melting" or "denaturation" of DNA will associate (sometimes referred to as reannealing or hybridization) reforming the duplexed structure. As is now well known in the art, contact of a first single stranded nucleic acid, either DNA or RNA, which comprises a base sequence sufficiently complementary to (i.e., "homologous with") a second single stranded nucleic acid under appropriate solution conditions, will result in the formation of DNA/DNA, RNA/DNA, or RNA/RNA hybrids, as the case may be.

In accordance with the present invention, a single stranded DNA is selected as an analytical reagent on the basis that it comprises a base sequence which is homologous, i.e., sufficiently complementary, with RNA found in substantially all bacteria of interest. Thus, hybridization between this DNA probe and RNA released in single stranded form from the test sample indicates the presence of bacteria in the sample.

The bacteria of interest will vary from one analytical situation to another. Through experimentation or experience in the field it will be known which bacterial species could be present in a particular test sample. One then can select from a variety of methods to isolate one or more probes containing homologous base sequences from the nucleic acid content of such bacteria species. The homologous base sequence need not be a single continuous polynucleotide segment, but may be comprised of two or more individual segments interrupted by nonhomologous sequences. These nonhomologous sequences may be linear, or they may be self-complementary and form hairpin loops. In addition, the homologous region of the probe may be flanked at the 3'- and 5'-terminii by nonhomologous sequences, much as those comprising the DNA or RNA of a vector into which the homologous sequence had been inserted for propagation. In either instance, the probe as presented as an analytical reagent will exhibit detectable hybridization at one or more points with sample nucleic acids of interest. Linear or circular single stranded polynucleotides can be used as the probe element, with major or minor portions being duplexed with a complementary polynucleotide strand or strands, provided that the critical homologous segment or segments are in single stranded form and available for hybridization with sample DNA or RNA. Particularly preferred will be linear or circular probes wherein the homologous probe sequence is in essentially only single stranded form.

Homologous base sequences for the bacteria of interest are obtainable in several manners. A particularly important method for obtaining a homologous sequence involves selection of a gene or segment thereof which is known or experimentally determined to be conserved across substantially all bacterial species of interest. Such selected gene can be structural or regulatory, or perform some other function. If a structural gene, it need not be expressed in some or all of the bacterial species of interest since the present method is based not on expression but rather on the presence of hybridizable RNA.

The selected gene will usually comprise a structural gene for an expressed protein or nucleic acid. It has been particularly found that broad spectrum bacterial detection capability is provided where the homologous base sequence comprises at least a portion of one of the strands, i.e., either the sense or anti-sense strand, of a gene which codes for the synthesis of a nucleic acid or protein comprised in the mechanism of protein synthesis in the cell. Fundamentally, such mechanism involves the processes of transcription and translation. Transcription will be understood to define the process by which the genetic code in DNA is converted to messenger RNA (mRNA). Translation will be understood to define the process by which the nucleotide sequence of mRNA is translated into the amino acid sequence of a synthesized protein.

Therefore, in a preferred embodiment the homologous base sequence is obtained from a gene which codes for a nucleic acid or protein involved in the mechanisms of transcription or translation. In the case of transcription, such nucleic acids and proteins include the RNA polymerases, protein initiator factors (sigma factor), and protein termination factors (rho factor). In the case of translation, included are those nucleic acids and proteins involved in the three fundamental stages of protein synthesis - initiation, elongation, and termination. Thus, such materials include transfer RNAs (tRNA- comprising the functionally distinct classes for each of the 20 fundamental amino acid building blocks), ribosomal RNAs (rRNA - comprising the various known fragments and subunits such as the 5S, 16S, 23S, and 40S elements), ribosomal proteins, aminoacyl-tRNA synthetases, initiation factors (including IF-1, IF-2, and IF-3), elongation factors (including EF-1, EF-2 EF-G, and EF-Tu), and releasing factors. It should be understood that mRNA is not included in the above-described preferred class since the genes which code for mRNA synthesis will vary according to the trait for which the gene codes. Thus, for the purposes of this invention, mRNA as such is not part of the mechanism of protein synthesis but rather can be considered as the variable informational input to an otherwise essentially constant chemical system.

In addition to the above preferred class of genes from which to select the probe of the present invention, one can also choose from genes coding for other proteins and nucleic acids. Some examples are genes coding for such materials as various RNAs other than those involved in protein synthesis, such as 5S-RNA and priming RNAs for DNA replication; genes coding for bacterial membrane proteins such as membrane lipo-proteins from Gram-negative bacteria; genes coding for insertion sequences; transposons; and the like.

One strategy for development of a probe is to begin with cloned DNA which codes for ribosomal RNA. Ribosomal DNA has been cloned and sequenced for several species of plants, animals and bacteria including *E. coli*.

The 5S RNAs are 115 to 120 nucleotides long and have a high degree of sequence identity between prokaryotic and eucaryotic organisms. Probes based on 5S RNA would be useful for detection of bacteria in samples which do not contain significant eucaryotic cellular products.

16S Ribosomal RNA from *E. coli* is 1542 bases long and has a sequence nearly identical to those of other gram negative bacteria such as *Proteus vulgaris*. There is a high degree of homology between 16S ribosomal RNAs from *E. coli* and *Bacillus brevis*. Therefore, the *E. coli* sequence could also be used to detect gram positive bacteria.

In analyzing clinical samples for bacteria, it is desirable to differentiate between 16S bacterial RNA and mammalian 18S RNA and mitochondrial RNAs. This goal can be realized because the RNAs differ substantially in length and the identity among sequences from various species of bacteria is much greater than between bacterial and mammalian sequences. If necessary, a probe based on 16S RNA can be made more specific for bacteria by selecting certain portions of the sequence. For example, the 3'-hydroxyl terminus of mouse 18S RNA has segments which are very homologous with the 3'-terminus of *E. coli* 16S RNA. Elimination of a segment, for example, 230 bases, at the 3'-terminus of a 16S RNA probe could be used to improve the selectivity for bacteria over mammalian cells.

The 23S ribosomal RNA from *E. coli* is 2904 nucleotides long and the corresponding 28S RNA from the rat is larger, 4718 nucleotides. The rat sequence has a high degree of identity with sequences from other higher organisms such as the frog (75% identity) and less identity with sequences from lower organisms such as yeast (about 50% identity). A probe which codes for 23S RNA from *E. coli* will thus be useful for detection of bacteria in clinical specimens which may contain mammalian cells.

Another useful method for obtaining homologous base sequences for bacteria of analytical interest involves experimental screening of the nucleic acid content of such bacteria. This empirical method can be accomplished by a variety of schemes.

In one such scheme, ribosomal RNA is isolated from one of the *n* bacterial strains of interest designated strain A and separated into size classes of 5S, 16S and 23S RNA by sucrose gradient centrifugation. The RNA is labelled with $\alpha^{32}$P-ATP by using *E. coli* Poly A Polymerase. The DNA from all the bacterial strains of interest is isolated and fragmented by restriction endonuclease digestion. These DNA fragments are separated according to size by agarose gel electrophosesis and the DNA fragments are transferred to nitrocellulose by blotting. These DNA fragments on the nitrocellulose are then probed with the labeled RNA. If any of the probes from 5S. 16S or 23S RNA hybridize to some of the DNA fragments from all the strains tested this indicates that there exists a common DNA sequence among all the strains. In order to enrich for and isolate this DNA sequence, the DNA restriction fragments of either strain B, C, D or E are separated by chromatography on an ion exchange resin (such as RPC-5). This procedure separates DNA restriction fragments on the basis of size, AT content and structural features. The fractions from ion exchange chromatography are analyzed by agarose gel electrophoresis and blotted onto nitrocellulose. The nitrocellulose sheet is exposed to the ribosomal RNA probe to determine which fraction of DNA from the ion

5

exchange chromatography is enriched for the common sequence shared with strain A. The DNA fractions enriched for ribosomal DNA are cloned and the clones screened for the presence of the ribosomal DNA. This DNA can then be processed by conventional techniques to yield polynucleotide probes useful in the present invention.

In another scheme, RNA is isolated from one of the *n* bacterial strains of interest and appropriately labeled (e.g., with $\alpha^{32}$P-ATP using *E. coli* Poly A polymerase). The labeled RNA from strain A are then mixed with a sample of heat denatured DNA from strain B and resulting DNA/labeled RNA hybrids are separated from remaining nucleic acids. The separated hybrids, representing homologous sequences in the mRNA and tRNA from strain A to the DNA from strain B, are heat denatured and mixed with a sample of heat denatured DNA from strain C. This is repeated for all *n* strains and the final DNA/labeled RNA hybrids will contain RNA sequences that are enriched for homology to a sequence of DNA that is present in all of the strains tested. Such hybrids can then be processed by conventional techniques to yield polynucleotide probes useful in the present invention.

Having selected or experimentally obtained a suitable polynucleotide probe, reagent quantities can be prepared by conventional cloning techniques in suitable vectors such as pBR322, pHV33, pUC9, Lambda and derivatives thereof, and pYIP12. A particularly preferred vector is M13 bacteriophage (and derivatives) since only single stranded DNA probe will be generated, thereby eliminating the need to denature the probe prior to use in the assay and preventing self-hybridization [*Molecular Cloning, A Laboratory Manual*, T. Maniatis et al, Cold Spring Harbor Laboratory (1982) pp. 51 *et seq*].

The extent and specificity of nucleic acid reassociation is affected by:

1. The purity of the nucleic acid preparation.

2. Base composition of the probe - G-C base pairs will exhibit greater thermal stability than A-T base pairs. Thus, hybridizations involving higher G-C content will be stable at higher temperatures.

3. Length of homologous base sequence - Any short sequence of bases (e.g., less than 6 bases), has a high degree of probability of being present in many nucleic acids. Thus, little or no specificity can be attained in hybridizations involving such short sequences. The present homologous probe sequence will be at least 10 bases, usually 20 bases or more, and preferably greater than 100 bases. From a practical standpoint, the homologous probe sequence will often be between 300-1000 nucleotides.

4. Ionic strength - The rate of reannealing increases as the ionic strength of the incubation solution increases. Thermal stability of hybrids also increases.

5. Incubation temperature - Optimal reannealing occurs at a temperature about 25-30° C below the melting temperature (Tm) for a given duplex. Incubation at temperatures significantly below the optimum allows less related base sequences to hybridize.

6. Nucleic acid concentration Normally, to drive the reaction towards hybridi-zation, one of the hybridizable sample nucleic acid or probe nucleic acid will be present in excess, usually 100 fold excess or greater.

7. Denaturing reagents - The presence of hydrogen bond disrupting agents such as formamide and urea increases the stringency of hybridization.

8. Incubation time - The longer the incubation time the more complete will be the hybridization. Normally, incubation times are between 6 and 24 hours.

9. Volume exclusion agents - The presence of these agents, as exemplified by dextran and dextran sulfate, are thought to effectively increase the concentration of the hybridizing elements thereby increasing the rate of resulting hybridization.

Based on the above criteria and related factors known in the art, contact between the probe and denatured sample nucleic acids will be accomplished under conditions favorable to hybridization. As conditions are made more stringent, greater complementarity is required for formation of probe hybrids. Hybridization conditions of low salt and high temperature increase the stringency of annealing. One of the most popular conditions for hybridization is in 2XSSC (where 1XSSC = 0.15M sodium chloride and 0.015M sodium citrate, pH 7.0) at 65° C. Under such conditions, hybridization generally will require at least about 15-20 contiguous, exactly matched base pairs in the probe.

In some applications, it may be desirable to allow hybridization between less complementary sequences, e.g., to allow for evolutionary mutations affecting nucleic acid sequence but not overall cellular function and structure. The ability of related but not identical complementary sequences to reanneal can be controlled by the conditions of stringency. High stringency, e.g., elevated temperatures, requires formation of greater numbers of base pairs in a given length of duplex since reassociation is restricted to closely related sequences. At lower temperatures, i.e., less stringency, more distantly related sequences can reanneal. Additionally, increasing incubation time also permits less related sequences to anneal.

Practice of the present analytical method is not limited to any particular hybridization format. The manner in which hybridization resulting between the probe and sample nucleic acids is determined is primarily a matter of convenience. Any conventional hybridization technique can be used. As improvements are made and as conceptually new formats are developed, such can be readily applied to carrying out the present method.

Conventional hybridization formats which are particularly useful include those wherein the sample nucleic acids or the polynucleotide probe is immobilized on a solid support (solid-phase hybridization) and those wherein the polynucleotide species are all in solution (solution hybridization).

A. Solid-phase Hybridization

In this approach, one of the polynucleotide species participating in hybridization is fixed in an appropriate manner in its single stranded form to a solid support. Useful solid supports are well known in the art and include those which bind nucleic acids either covalently or noncovalently. Noncovalent supports which are generally understood to involve hydrophobic bonding include naturally occurring and synthetic polymeric materials, such as nitrocellulose, derivatized nylon, and fluorinated polyhydrocarbons, in a variety of forms such as filters or solid sheets. Covalent binding supports are also useful and comprise materials having chemically reactive groups or groups, such as dichlorotriazine, diazobenzyloxymethyl, and the like, which can be activated for binding to polynucleotides.

A typical solid-phase hybridization technique begins with immobilization of sample nucleic acids onto the support in single stranded form. This initial step essentially prevents reannealing of complementary strands from the sample and can be used as a means for concentrating sample material on the support for enhanced detectability. The polynucleotide probe is then contacted, in a single stranded, labeled forms with the support. Appropriate labels are available by which to detect resulting hybridization on the support. Typically, a solid-phase hybridization technique will proceed as follows:

(1) the test sample is subjected to conditions to release and denature bacterial RNA and resulting single stranded RNA is immobilized on a solid support, e.g., a liquid sample such as a body fluid is applied. to a nitrocellulose membrane, the deposited cells are lysed and released RNA denatured, and the membrane is baked in vacuo at 80°C for 2 hours to fix single stranded RNA to the membrane; alternatively, the cells are first lysed and released RNA is denatured and then applied to the nitrocellulose membrane;

(2) the support is contacted with the probe in excess under favorable hybridization conditions, e.g., after saturating all nonspecific binding sites on the membrane by treatment at 40-60°C with a prehybridization solution comprising buffer (e.g., 2XSSC), protein such as bovine serum albumin, Ficoll® (a trademark identifying a copolymer of sucrose and epichlorohydrin sold by Pharmacia Fine Chemicals, Piscataway, NJ), polyvinylpyrrolidone, and a denatured foreign DNA such as from calf thymus or salmon sperm; typically the hybridization conditions will be the same as the prehybridization condition except the time of incubation will usually be longer;

(3) removing probe which has not become associated with the support by hybridization, e.g., by simple washing of the membrane; and

(4) measuring on the support.

Additional steps may also be included in the above typical protocol. For example, where particularly short DNA fragments (e.g., less than about 1000 bases) are to be immobilized, such polynucleotides can be first derivatized with glyoxal and then applied to the support. Alternatively, reactive cellulose can be used to covalently bind the polynucleotides, usually after an initial purification of the sample to isolate nucleic acids according to standard methods.

An example of a hybridization format using covalent binding, chemically derivatized paper, such as diazobenzyloxymethyl cellulose, is as follows:

(1) release and purify RNA from bacteria present in the sample,

(2) denature the sample RNA by heating at 100°C for several minutes,

(3) spot the denatured mixture onto the chemically activated paper,

(4) allow to air dry, then store for 12-16 hours in a closed container,

(5) wash the paper with water, 0.4N sodium hydroxide, and again with water,

(6) rinse the paper with prehybridization buffer,

(7) incubate the paper for 1 hour in an elution buffer [e.g., 99 % (v/v) deionized formamide in 10 mM Tris. HCl buffer (pH 7.8)],

(8) wash the paper again with prehybridization solution,

(9) add DNA probe in hybridization solution,

(10) wash the paper to remove excess, unhybridized probe, and

(11) measure on the paper.

The assay method use a DNA probe which is complementary to a conserved sequence of an RNA such as 5S, 16S, or 23S RNA. The probe could be immobilized on a solid support and hybridized with the RNA from the sample, and the RNA/DNA duplex would be detected in any convenient manner, such as with labeled antibody specific for RNA/DNA hybrids [see Rudkin and Stollar, *Nature 265*:472 & 473 (1977), Stuart *et al*, *PNAS 78*:3751 (1981), Reddy and Sofer, *BBRC 103*:959-967 (1981), and Nakazato, *Biochem. 19*: 2835-2840 (1980)]. The use of immobilized DNA probes for detecting the presence of RNA sequences is possible because of the ability to prepare antibodies which are selective for DNA•RNA hybrids over the single stranded polynucleotides as is known in the art. Specific binding antibody fragments such as Fab and Fab' can also be used. Determination of binding of antibody to hybridization duplexes can be accomplished in any convenient manner, preferably by using an antibody or fragment thereof which is labeled with a detectable chemical group such as an enzymatically active group (e.g., an enzyme, an enzyme substrate, a coenzyme, or an enzyme inhibitor), a luminescer, a specifically bindable ligand (e.g. a hapten or biotin), a fluorescer, a chromophore, or a radioactive isotope, as is more fully described below. Alternatively, the antibody can be detected based on a native property such as its own antigenicity. A labeled anti(antibody) antibody will bind to the primary antibody reagent where the label for the second antibody is a conventional label as above. Further, antibody can be detected by complement fixation or the use of labeled protein A, as well as other techniques known in the art for detecting antibodies.

This assay method has the advantage that the probe can be immobilized, thus the analyst does not have to immobilize the sample RNA prior to the hybridization step. Sometimes immobilization of sample nucleic acids can be problematical due to interferences by proteins and other materials in the sample. A further advantage is that each cell contains thousands of ribosomes. As a result, the detection limit is greatly improved over methods which detect genomic sequences. During hybridization of cellular RNAs with probes, it may be necessary in certain instances to protect the RNAs from degradation by ribonucleases. These enzymes can be effectively inhibited by a variety of substances such as heparin, sodium dodecyl sulfate, ribonucleoside-vanadyl complexes, aurintricarboxylic acid and dextran sulfate.

B. Solution, Hybridization

The present method can also be used for detection of bacterial nucleic acids in a solution format. This normally requires that the homologous DNA sequence be in single stranded form. This will be referred to as the probe polynucleotide.

In a solution format, the specimen nucleic acids are first released from bacterial cells in the sample by lysis, and then denatured. These steps may be combined by heating the sample to 100° C or by exposing it to base. After adding a solution containing a large excess of the probe, hybridization is allowed to occur under conditions of ionic strength and temperature empirically determined to give the desired probe specificity and sensitivity.

Hybrids can be detected and quantitated using the above method.

A technique not requiring incorporation of a label into a polynucleotide probe has been described by Rudkin and Stollar [*Nature 265*:472-473(1977)]and used by cytochemists for detection of hybridization *in situ*. In this approach, single stranded RNA containing the desired sequence is hybridized to DNA from the sample. Hybridization is detected using labeled antibodies specific for RNA/DNA hybrids (these antibodies do not bind to double stranded DNA or single stranded RNA or DNA). This approach is applicable to the present method. Preferred antibody labels would be those described above.

In general, labels and labeling approaches which have been developed for use in immunoassays will be applicable to the present hybridization technique with modifications evident to the ordinary skilled worker. See U.S. Pat. Nos. 4,380,580; 4,279,992; 4,238,565; 4,134,792; 4,273,866; 3,817,837; 4,043,872; 4,238,195; 3,935,074; 3,998,943; 3,654,090; 3,992,631; 4,160,016; and 3,996,345; British Pat. Specs. 1,552,607 and 3,019,408; and European Pat. Applns. 70,687; 70,685; and 63,879; the pertinent parts of which relating to labeling schemes are incorporated herein by reference.

The test sample to be assayed for bacterial presence can be any medium of interest, and will usually be a liquid sample of medical, veterinary, environmental, nutritional, or industrial significance. Human and animal specimens and body fluids particularly can be assayed by the present method, including urine, blood (serum or plasma), milk, cerebrospinal fluid, sputum, fecal matter, lung aspirates, throat swabs, genital swabs and exudates, rectal swabs, and nasopharyngeal aspirates. The present invention has particular advantages for the detection of bacteria in urine (bacteriuria). The detection of $10^5$ or more bacterial colony forming units per milliliter in a urine sample is presumptive of a urinary tract infection. In uncomplicated infections, the etiological agents comprise two general categories according to incidence:

Category 1 (90% incidence)

Family: *Enterobacteriaceae (Gram-negative*
*facultatively anaerobic rods)*
*Eacherichia (E. coli)*
*Klebsiella (K. pneumoniae)*
*Enterobacter (E. cloacae, E. aerogenes)*
*Proteus (P. vulgaris, P. mirabilis,*
*Morganella morganii)*

Category 2 (5-10% incidence)

Family: *Pseudomonadaceae* (Gram-negative aerobic rods and cocci)
*Pseudomonas (P. aeruginosa)*
Family: *Micrococcaceae* (Gram-positive cocci)
*Staphylococcus (S. aureus)*
Family: *Streptococcaceae* (Gram-positive cocci)
*Streptococcus Group D (Enterococci)*

In complicated infections, strains of *Citrobacter, Serratia,* and *Providencia* also may appear. Probes homologous for this entire group or subsets thereof can be obtained according to the present invention depending on the degree of specificity and sensitivity desired for a particular bacteria test.

While the present invention has been particularly described with reference to, and is most advantageously applied to, the detection of bacteria, it will be understood that this method can be used to detect other types of microorganisms such as protozoa, yeasts, viruses, and so forth.

The test sample can be treated in a variety of known manners in order to release bacterial nucleic acids in single stranded form. Release of nucleic acids can, for example, be obtained by mechanical disruption (freeze/thaw, abrasion, sonication), physical/chemical disruption (detergents such, as Triton, Tween, sodium dodecylsulfate, alkali treatment, osmotic shock, heat-boiling water), or enzymatic lysis (lysozyme, proteinase K, pepsin). Denaturation of released nucleic acids is preferably accomplished by heating in boiling water or alkali treatment (e.g., 0.1 N sodium hydroxide), which, if desired, can simultaneously be used to lyse the cells.

The present invention additionally provides a reagent system, i.e., reagent combination or means, comprising all of the essential elements required to conduct a desired assay method. The reagent system is presented in a commercially packaged form, as a composition or admixture where the compatibility of the reagents will allow, in a test device configuration, or as a test kit, i.e., a packaged combination of one or more containers, devices, or the like holding the necessary reagents, and usually including written instructions for the performance of assays. Reagent systems of the present invention include all configurations and compositions for performing the various hybridization formats described herein. Particularly preferred is a test kit for performing a solid-phase hybridization protocol comprising (1) a solid support capable of immobilizing single stranded nucleic acid resulting from treatment of a test sample to release and denature bacterial nucleic acids, and (2) a labeled polynucleotide probe selected from the various types described herein. Preferably, such kit will additionally comprise foreign nucleic acid for use in substantially saturating nucleic acid adsorption sites on the support after immobilization of sample nucleic acid, along with, optionally, other desired ingredients for a prehybridization solution. Also, the kit will preferably include a chemical lysing and denaturing agent, e.g., alkali, for treating the sample to release single stranded nucleic acid therefrom. Ingredients for the hybridization reaction, if different from the prehybridization solution, will also be preferably included in the kit. The reagent system can, of course, include other materials and solutions as are known in the art and which may be desirable from a commercial and user standpoint, such as buffers, diluents, standards, and so forth.

The present invention will now be illustrated, but is not intended to be limited, by the following examples.

EXAMPLE 1

A. *Growth of microorganisms used as test strains.*

The organism *Escherichia coli* No. 063 (University of Rochester Medical Center, Rochester, NY) was obtained from the urine of a patient that tested positive for urinary tract infection. As a control non-urinary tract infection isolate, the microorganism *Bacillus subtilis* (No. 1A289 from the Bacillus Genetic Stock Center, Ohio State University, Columbus, OH) was used. Each organism was grown in a liquid nutrient medium in the following manner.

Each shake flask was inoculated with either *E. coli* or *B. subtilis.* The shake flasks contained fifty milliliter (ml) of 2XYT broth. 2XYT consists of 1.6% Bactotryptone, 1.0% yeast extract and 0.5% sodium chloride. Bactotryptone and yeast extract are available from Difco Laboratories, Detroit, MI. Both cultures were grown at 37° C in a rotary shaker for 6 hours.

In order to determine the bacterial count per unit volume of each 6 hour broth culture, the culture was serially diluted by ten-fold increments into 2XYT broth. Each of the dilutions were plated on sterile agar medium containing tryptose blood agar base (TBAB) and 0.1% casamino acids (both components available from Difco). Those plates were incubated at 37° C for 12 hours to allow colonies to form. The plates containing 30-300 colonies were counted and the number of cells per unit volume in the original broth cultures was determined by calculation.

B. *Application of cells from broth culture to a solid support for immobilization of nucleic acids.*

The six-hour broth cultures and freshly made dilutions of these cultures were applied to a solid support consisting of nitrocellulose (Bio-Rad Transblot® transfer medium available from Bio-Rad Laboratories, Richmond, CA). The addition of a precise number of cells to a constant surface area of the nitrocellulose was accomplished by the use of an apparatus called a Minifold® which is commercially available from Schleicher & Schuell, Inc., Keene, NH. This apparatus is a filter manifold consisting of three sections which are clamped together into a "sandwich" during the filtration or immobilization process. The MinifoldTM apparatus was fitted with one 4 inch (") x 5 1/4" sheet of the nitrocellulose membrane, backed by two 4" x 5 1/4" sheets of Whatman 3 MM chromatography paper (available from Whatman Ltd., England). The nitrocellulose and Whatman paper were presoaked in five ml of 1XSSC (1XSSC = 0.15M NaCl, 0.015M trisodium citrate). The membrane and backing papers were clamped into the apparatus. The apparatus was hooked up to a vacuum line and the vacuum applied. To each well was added 0.10 ml of the 6 hour *E. Coli* culture and to successive wells, 0.10 ml the serial dilutions of this culture. The control 6 hour culture of *B. subtilis* was also collected on the filter in the same fashion as described for the *E. coli* culture. As an additional control, nutrient media was added to another set of wells.

C. *Lysis of cells and denaturation of DNA on membrane containing the cells.*

The nitrocellulose membrane containing the cells was removed from the Minifold® apparatus and placed on a Whatman 3MM paper which had been saturated with a 0.5M sodium hydroxide solution. The membrane was placed on paper so the cells were always on the upper surface. The nitrocellulose membrane remained in contact with the 3MM paper for 10 minutes at 65° C. The nitrocellulose membrane was then placed on a Whatman 3MM paper that had been presoaked with 1M Tris, pH 7.4. The nitrocellulose remained in contact with the paper for 10 minutes at 65° C. The nitrocellulose membrane was removed and placed on a second 3MM paper saturated with 1M Tris, pH. 7.4, For 10 minutes at 65° C. The nitrocellulose was removed and placed on a Whatman 3MM paper saturated with a solution of 0.5M Tris, 1.5M NaCl, pH 7.4, for 10 minutes at 65° C. The nitrocellulose membrane was then air dried and baked at 80° C for 2 hours in a vacuum oven.

In a typical experiment, samples containing $1 \times 10^8$, $1 \times 10^7$, $1 \times 10^6$, $1 \times 10^5$, $1 \times 10^4$, $1 \times 10^3$, $1 \times 10^2$, $1 \times 10^1$ cells were applied to each well.

D. *Isolation and purification of M13-10 replicative form DNA.*

The bacteriophage M13-10 (ATCC 39403-B1] was used as the source of the *tuf* A gene fragment. The *tuf* A gene of *E. coli* encodes for the protein EF-Tu. EF-Tu is a single polypeptide chain with a molecular weight of 43,225. The EF-Tu protein is an elongation factor in protein synthesis and mediates the entry of an aminoacyl-tRNA into the A site of the ribosome. The EF-Tu protein carries a guanine nucleotide (GTP). The EF-Tu-GTP binds aminoacyl-tRNA. The size of the *tuf* A gene is 1,190 base pairs. The portion of the *tuf* A gene contained in M13-10 is an 800 base fragment. The *tuf* A gene is cloned between the Hinc II and EcoRI restriction endonuclease sites of the vector M13mp9 (New England BioLabs, Beverly, MA).

The *Escherichia coli* host organism used for growth of M13-10 phage was JM103 (Δ*lac, pro) supE. thi, strA, endA, sbcB15, hsdR4, traD36, lac IqZM13.* This strain is commercially available from Bethesda Research Laboratories, Gaithersburg, MD.

Fifteen ml of an overnight culture of the above *E. coli* was inoculated into one liter of 2XYT broth in a two liter Fernbach flask. This culture was grown for 3 hours at 37°C on a New Brunswick Gyrotary® shaker (200 rpm). To the 3 hour culture 2 x 10¹¹ PFU (plaque forming units) of M13-10 bacteriophage were added. This infected culture was allowed to incubate for 1 hour under the same conditions as described above. At this time, 150 mg of chloramphenicol was added to the culture. Incubation was continued for an additional 45 minutes. Cells were harvested by centrifugation (GSA rotor, 5,000 rpm, 10 minutes) and washed once in cold 2XYT broth. The washed cells were suspended in 40 ml cold 10% sucrose solution. Lysis was accomplished by the addition of 20 mg of lysozyme and 16 ml of 0.25M ethylenediaminetetraacetic acid (EDTA) followed by incubation in an ice bath for 10 minutes. Lysis was completed by the addition of 4 ml 20% sodium dodecyl sulfate (SDS). The lysis mixture was mixed gently. The concentration of sodium chloride in the lysate solution was adjusted to 1M with the addition of 2.90 gm of sodium chloride. The lysate mixture was incubated at 40°C overnight. The lysate was cleared by 4°C centrifugation in a Beckman ultracentrifuge at 60,000 xg for 30 minutes. The supernatant was extracted three times with equal volumes of a phenol-chloroform mixture composed of 4 parts of phenol-1 part of chloroform. The aqueous phase from the extraction was added to two volumes of cold 95% ethyl alcohol. This mixture was incubated at -70°C for thirty minutes and then centrifuged 1,800 xg for 15 minutes at 4°C. The pellet that resulted was washed with 70% ethyl alcohol and then air dried. The DNA pellet was dissolved in 15 ml of a solution containing 0.01M Tris, pH 8, 0.001M EDTA. This DNA solution was subjected to isopycnic cesium chloride density gradient centrifugation in the presence of ethidium bromide to separate covalently closed circular replicative form DNA from linear DNA. Cesium chloride was added to the DNA solution at ambient temperature to a final refractive index of 1.3930. After centrifugation for 40 hours at 130,000 xg, the lower band containing the replicative form DNA was removed and extracted 6 times with water-saturated sec-butanol to remove ethidium bromide. The extract was dialyzed extensively against TE buffer (0.01M Tris, pH 8.0, 0.001M EDTA) at 4°C. To remove contaminating RNA, 250 mg of ribonuclease (RNase) was added per ml of dialysate and the solution incubated for 1 hour at 37°C. To remove the RNase, the solution was extracted with an equal volume of phenol saturated with 0-1M Tris, pH 8, buffer. The upper phase from the phenol extraction was made 0.3M in sodium citrate and precipitated with 2 volumes of cold ethanol at -70°C for 30 minutes. The phage DNA was dissolved in, TE buffer.

The M13-10 replicative form DNA was then cleaved by a double digest with *Sal I, EcoRI* under appropriate conditions such that the 800 bp (base pair) insert of the *tuf A* gene fragment was cut out of the vector.

### E. *Isolation and radioactive labeling of the tuf A fragment.*

The double digested *Sal I, EcoRI* M13-10 DNA was adjusted to a concentration of 40 μg per 100 μl. Horizontal agarose gel electrophoresis was performed in a Bio-Rad Sub-Cell® electrophoresis chamber (available from Bio-Rad Laboratories), A 1 percent low gelling temperature (LGT) agarose gel (available from Miles Research Products, Naperville, IL) in 0.089M Tris borate, 0.089M boric acid, 0.002M EDTA (TBE), pH 8, buffer containing 0.5 μg/ml ethidium bromide was formed in the Sub-Cell apparatus. The M13-10 double digested DNA solution was loaded on this gel at a concentration of 7 μg per well. Electrophoresis was performed at 50 volts for 3 hours at ambient temperature. The insert *tuf A* fragment DNA was cut out of the gel. These gel pieces were placed in a 50 ml polypropylene tube and heated to 70°C until the gel melted. The molten gel was adjusted to a total volume of 10 ml with 0.2M NaCl, 0.02M Tris, pH 7.0, 0.001M EDTA. The DNA was isolated from this gel solution through the use of the Elutip®d columns commercially available from Schleicher & Schuell, Keene, NH. The procedure used was that recommended by the supplier. The Elutip®d column was washed with 2 ml of a solution of 1.0M NaCl,0.02M Tris-HCl, pH 7.3, 0.001M ETDA. The column was washed with 0.5 ml of a solution comprised of 0.2M NaCl, 0.02M Tris-HCl, pH 6.3, 0.001M ETDA. A 0.45 μm cellulose acetate disposable filter was attached to the syringe (available from Schleicher & Schuell) and the DNA sample loaded onto the column. The DNA adsorbs to the matrix of the column. The column was washed with 3 ml of a solution containing 0.2M NaCl, 0.02M Tris-HCl, pH 7.3, 0.001M EDTA. Elution of the DNA sample from the column was accomplished by removing the cellulose acetate filter and adding 0.4 ml of the solution containing 1M NaCl, 0.02M Tris-HCl, pH 6.3, 0.001M EDTA. The DNA was precipitated with cold ethanol and dissolved in 40 μl of water.

The *tuf A* gene fragment was labeled with ³²p by nick translation as described below. A reaction mixture was prepared consisting of 0.94 μg of *tuf A* DNA fragment, 20 micromoles each of deoxycytidine triphosphate (dCTP), deoxyguanosine triphosphate (dGTP) and deoxythymidine triphosphate (dTTP), 0.05M Tris-HCl, pH 7.8, 0.005M magnesium chloride, 0.01M 2-mercaptoethanol, 0.50 μg of nuclease free bovine serum albumin (BSA), 2 units of DNA polymerase I (available from BRL, Gaithersburg, MD) 200 picograms DNase I, 0.0005M magnesium acetate, 250 $\mu$g glycerol, and 4.88 $\mu$moles of ($\alpha^{32}$P] deoxyadenosine triphosphate (dATP) with a specific activity of 410 Ci/mmol. The total reaction volume was adjusted to 50 microliters with distilled water and incubated at 15°C for one hour. The reaction was stopped by the audition of 5 microliters of 0.3M Na$_2$EDTA (pH 8.0) and extracted with an equal volume of Tris-saturated phenol. The extracted mixture was chromatographed on a Sephadex G-50® column (Pharmacia Fine Chemicals, Piscataway, NJ) to remove the unreacted deoxytriphosphates. The $^{32}$P labeled DNA fragments were contained in a total volume of 400) microliters with a specific activity of 8 x 10$^7$ cpm per microgram of DNA.

## F. *Hybridization protocol*

The nitrocellulose membrane described in part B of this example was pretreated before hybridization by placing the membrane in a heat sealable bag (commercially available as Boil-A-Meal® bags from Sears, Roebuck & Co., Chicago, IL) and adding 10 ml of a solution consisting of 50% formamide (Matheson, Coleman & Bell), 5XSSPE (1XSSPE = 0.18M] NaCl, 0.01M sodium phosphate, 0.001M Na$_2$ EDTA, pH 7.0), 5XBFP (1XBFP = 0.02% w/v of bovine serum albumin, Ficoll [MW. 400,000] and polyvinyl pyrrolidone), 1% glycine, and 100 $\mu$g/ml of denatured, sonicated carrier salmon sperm DNA. The membrane was incubated in this solution for one hour at 42°C. The prehybridization solution was then removed. Four milliliters of a solution containing 50% v/v formamide, 5XSSPE, 1XBFP, 100 $\mu$g/ml sonicated salmon sperm DNA, 10% w/v sodium dextran sulfate 500 and 0.3% SDS was prepared. Half of this solution (2 ml) was added to the bag containing the membrane and mixed. The labeled probe DNA was denatured by heating the DNA in a solution of 10mM Tris (pH 7.5), 1mM EDTA for 10 minutes at 95°C and cooling it quickly in an ice bath. The denatured probe DNA was added to the remaining 2 ml of solution at a quantity sufficient to provide 5 x 10$^6$ cpm per membrane. The 2 ml of solution containing the probe was then added to the bag and the contents were mixed. The bag was sealed by using a heat-sealer.

The hybridization mixture was incubated for 20 hours at 42°C. The nitrocellulose membrane was removed from the hybridization solution and washed in a solution of 2XSSPE and 0.2% SDS. This was was repeated four times at 45°C. The membranes were air dried and placed in contact with X-ray film (Dupont Cronex 4) and placed in a film cassette fitted with a "Lightning Plus" intensifying screen (available from Dupont). The film was exposed to the X-ray film for an appropriate time (this varies depending on the specificity and specific activity of the probe DNA and rate of the hybridization reaction).

## G. *Detection of the presence of a bacteria that had been isolated from urine of a human patient with a urinary tract infection.*

In a typical experiment as in this example, the presence of 1X10$^6$ bacteria comprised of *Escherichia coli* No. 063 was detected using the *tuf A* gene fragment as a probe. The present of a Gram positive organism *Bacillus subtilis*, a non-urinary tract isolate, was not detected even when as many as 1X10$^8$ cells were present.

## EXAMPLE 2

### A. *Isolation and purification of DNA from bacterial strains that have been isolated from patients with urinary tract infections.*

When the test organism is of the family *Enterobacteriaceae*, genus and species *Escherichia coli, Proteus mirabilis, Enterobacter cloacae, Enterobacter aerogenes, Citrobacter freundii, Hafnia alvei, Klebsiella pneumoniae, Klebsiella oxytoca, Morganella morganni, Serratia rubideae, Providencia stuartii,* and *Klebsiella ozaenae,* and the Families *Streptococcaceae, Enterococcus, Micrococcaceae,* and *Staphylococcus aureus,* the procedure for the isolation of genomic DNA is essentially the same with the exception of *Staphylococcus aureus.*

The general procedure for isolation of genomic DNA Will be described for all the organisms named in the preceeding paragraph. Each of the aforesaid organisms was grown in 1 liter of TBB + CAA medium which is comprised of 1% tryptose, 0.3% beef extract, 0.1% casamino acids (available from Difco Laboratories) and 0.5% sodium chloride for 18 hours with aeration. Cells were harvested by centrifugation and washed in 75 ml of a solution of 0.15M NaCl and 0.015M trisodium citrate (1XSSC). The cells were suspended in a mixture of 25 ml of 1XSSC and 1.5 ml of 0.25M EDTA. Lysis and RNA degradation was accomplished by the addition of 12.5 mg of lysozyme (egg white crystalline lysozyme, commercially available from Miles Laboratories, Naperville, IL) and 7.5 mg RNAse followed by incubation for 1 hour at 37°C. A 0.7 ml volume of 25% SDS was added to the mixture and incubation was continued for 1 hour followed by the addition of 10 ml of distilled water and 5 ml of 5M sodium perchlorate. Protein was removed by extracting the lysate with 50 ml of chloroform isoamyl alcohol in a ratio of 24:1. The upper phase was removed, 10 mg of pronase added, and the mixture incubated for 2 hours at 37°C with gentle agitation. This mixture was extracted with 50 ml of chloroform-isoamyl alcohol The aqueous phase was extracted once more with chloroform-isoamyl alcohol. The DNA in the upper phase was precipitated with two volumes of cold 95% ethanol. The DNA was dissolved in sterile distilled water or 0.1 XSSC.

In the case of *Staphylococcus aureus,* lysis was accomplished by using Lysostaphin (available commercially from Sigma Chemical Co., St. Louis, MO), adding 375 $\mu$g of this enzyme to lyse biomass recovered from 1 liter of culture.

The DNA concentrations were determined using the fluorescence assay as described by A.R. Morgan *et al, Nucleic Acids Research 7*: 547 - 594(1979).

### B. *Immobilization of genomic DNA isolated from organisms onto a solid support.*

Samples of the DNA from each of the organisms named in Part A of this example and DNA from *Bacillus subtilis* and human placenta (human placental DNA is commercially available from Sigma Chemical Co., St. Louis, MO) were adjusted to DNA concentrations of 0.2 $\mu$g/$\mu$l, 0.2 $\mu$g/$\mu$l, 0.002 $\mu$g/$\mu$l, 0.20 ng/$\mu$l, 0.02 ng/$\mu$l and 2.0 pg/$\mu$l. These six DNA concentrations were applied to a solid support consisting of a Gene-Screen® membrane (New England Nuclear, Boston, MA). The application of a precise volume of DNA solution to a constant surface area of the membrane was accomplished by the use of the MinifoldTM filtration apparatus from Schleicher & Schuell, Inc., (see part B example 1). The Minifold apparatus was fitted with one 4" x 5 1/4" sheet of nitrocellulose membrane that had been prewetted in 1XSSC. Vacuum was applied to the Minifold apparatus and 5 microliters of each concentration of DNA from each of the organisms named above was added. After the membrane was air dried, the membrane was immersed in a denaturing solution consisting of 2.5M NaCl and 0.5M NaOH for 2 minutes, Subsequently, the membrane was immersed in neutralizing solution which consisted of 3M sodium acetate (pH 5.5). Excess solution was blotted from membrane with Whatman 3MM paper. The membrane was baked at 80°C in a vacuum oven for 1 hour.

### C. *Isolation and purification of M13-10 replicative form DNA.*

See part D, Example 1.

### D. *Isolation and radioactive labeling of the tuf A gene fragment.*

See part E, Example 1.

### E. *Hybridization protocol.*

See part F, Example 1.

### F. *Detection of the DNA of bacteria that comprise ninety-five percent of urinary tract infections in humans.*

In a typical experiment as in this example, the results were as shown in Table A.

Table A

| Family | Genomic DNA-Organism | # of Strains | Amount of DNA Detected |
|---|---|---|---|
| Enterobacteriaceae | Escherichia coli | 6 | 1.0 nanogram |
| " | Proteus mirabilis | 2 | 10.0 " |
| " | Enterobacter cloacae | 2 | 10.0 " |
| " | Enterobacter aerogenes | 1 | 1.0 " |
| " | Citrobacter freundii | 1 | 1.0 " |
| " | Hafnia alvei | 5 | 10.0 " |
| " | Klebsiella pneumoniae | 1 | 1.0 " |
| " | Klebsiella oxytoca | 2 | 10.0 " |
| " | Morganella morganii | 1 | 10.0 " |
| " | Serratia rubideae | 2 | 10.0 " |
| " | Providencia stuartii | 1 | 10.0 " |
| " | Klebsiella ozaenae | 1 | 10.0 " |
| Micrococcaceae | Staphylococcus aureus | 1 | * |
| Pseudomonadaceae | Pseudomonas aeruginosa | 2 | 1.0 " |
| Streptococcaceae | Enterococcus | 1 | .100.0 " |
| Bacillaceae | Bacillus subtilis | 1 | * |
|  | Human placental DNA | – | * |

* = not detectable

The results indicate that the *tuf A* gene probe is able to hybridize with essentially all of the organisms that are etiologic agents of urinary tract infections. The control DNA samples, *Bacillis subtilis,* a soil microorganism, and human DNA does not hybridize with the *tuf A* gene.

EXAMPLE 3

A. *Hybridization of DNA from urinary tract isolates lysed in situ on a nitrocellulose membrane filter.*

The following microorganisms were grown in TBAB (Tryptose Blood Agar Base) - Difco agar plates + casamino acids (CA) overnight to obtain single colony isolates:

*Escherichia coli* A5
*Escherichia coli* A6
*Klebsiella pneumoniae* B4
*Klebsiella pneumoniae* B5
*Proteus mirabilis* C2
*Enterobacter cloacae* D4
*Morganella morganii* C7

A single colony isolate from each type of micro-organism was used as inoculum for ten ml of TBAB + CA broth. These cultures were grown overnight at 37°C. Each culture was diluted $10^{-2}$ to $10^{-7}$ in TBAB + CA media at 4°C to stop all growth. Each dilution was plated on TBAB + CA agar plates to determine the number of viable organisms per ml of media. Also, one ml of each dilution was transferred to a siliconized tube. To each tube was added: 50 micro-liters lysozyme (10 mg/ml) dissolved in 10 mM Tris, 1 mM EDTA buffer, pH 8, 100 microliters of 1%, SDS dissolved in 0.2 N NaOH (sodium hydroxide), and 0.5 ml modified 20x SSPE which consists of 3.6 M NaCl, 0.2 M $NaH_2PO_4 \cdot H_2O$, 0.16 M NaOH and 0.020 M $Na_2EDTA$. The SSPE is modified by adding sufficient 0.2 N NaOH solution to adjust the pH of the 20x SSPE to 7.4. The purpose of this mixture is to disrupt and lyse the bacterial cells and denature the DNA that is released from the cells.

The entire lysed cell mixture samples from each cell type were applied to a solid nitrocellulose support following the method of part B, Example 1. To each well of the Minifold apparatus was added the entire volume of cell lysate (which contains 1 ml of broth containing the cells and dilutions plus lysis reagents) of each sample.

A control culture of mammalian cells at a concentration of $1 \times 10^7$ cells per ml (determined by hemocytometer count) and serial dilutions of these cells was also lysed and collected on the filter in the same manner as described for the cultures of microorganisms. Each sample and control was done in duplicate.

The nitrocellulose membrane described above in this example was pretreated before hybridization by placing the membrane in the heat sealable bag commercially available from Sears Roebuck & Co., and adding 10 ml of a solution consisting of 5XBFP (1XBFP c 0.02% w/v bovine serum albumin, Ficoll of M.W. 400,000, and polyvinylpyrrolidone), 5XSSPE, 0.3% SDS, and 100 $\mu$g/ml salmon sperm DNA. The membrane was incubated in this solution for one hour at 70°C and the prehybridization solution removed. Ten milliliters of a solution containing 5XSSPE, 1XBFP, 100 $\mu$g/ml salmon sperm DNA and 0.3% SDS was prepared. Half of this solution (5 ml) was added to the bag containing the membrane and mixed. The labeled probe DNA (*tuf* fragment from part E, Example 1) was denatured by heating the DNA in a solution of 10 mM Tris (pH 7.5), 1 mM EDTA for 10 min. at 95°C and cooling it quickly in an ice bath. The denatured probe DNA was added to the remaining 5 ml of solution at a quantity sufficient to provide $5 \times 10^6$ cpm per membrane. The solution containing the probe was added to the bag and the bag was heat sealed.

The hybridization mixture was incubated for 16 hours at 70°C. The nitrocellulose membrane was removed from the hybridization solution and washed in a solution of 5XSSPE and 0.5% SDS. This was was repeated four times at 70°C. The membranes were air dried; subsequently, the membranes were cut up in such a manner as to cut out the filter area encompassed by each well of the Minifold as well as an equivalent border area for each well. These filter squares were placed into individual scintillation vials containing 5 ml of Aquasol® fluor (New England Nuclear, Villerica, MA). Duplicate samples of each cell type and its' serial dilutions were counted on a Beckman scintillation counter.

The results of this experiment are reported in Table B.

## Table B

| Cell Type | Number of Viable Cells Applied to Each Well of M Minifold | $^{32}$P-CPM-X Counts Per Minute-Measure of Extent of Hybridization of tuf Gene to DNA of Organism | Standard Deviation CPM |
|---|---|---|---|
| Escherichia coli #057 | $1.5 \times 10^{8}$ | 2,339 | 235 |
| | $1.5 \times 10^{7}$ | 699 | 122 |
| | $1.5 \times 10^{6}$ | 648 | 64 |
| | $1.5 \times 10^{5}$ | 114 | 11 |
| Escherichia coli #163 | $1.7 \times 10^{8}$ | 4,181 | 676 |
| | $1.7 \times 10^{7}$ | 1,252 | 304 |
| | $1.7 \times 10^{6}$ | 676 | 106 |
| | $1.7 \times 10^{5}$ | 118 | 14 |
| Klebsiella pneumoniae p. | $1.1 \times 10^{8}$ | 3,062 | 536 |
| | $1.1 \times 10^{7}$ | 670 | 111 |
| | $1.1 \times 10^{6}$ | 494 | 114 |
| | $1.1 \times 10^{5}$ | 102 | 3 |
| Klebsiella pneumoniae | $1.0 \times 10^{8}$ | 1,509 | 160 |
| | $1.0 \times 10^{7}$ | 430 | 59 |
| | $1.0 \times 10^{6}$ | 111 | 12 |
| | $1.0 \times 10^{5}$ | 98 | 5 |
| Proteus mirabilis | $2.0 \times 10^{8}$ | 2,677 | 633 |
| | $2.0 \times 10^{7}$ | 699 | 122 |
| | $2.0 \times 10^{6}$ | 647 | 64 |
| | $2.0 \times 10^{5}$ | 114 | 11 |
| Enterobacter cloacae | $5.0 \times 10^{7}$ | 1,341 | 71 |
| | $5.0 \times 10^{6}$ | 315 | 15 |
| | $5.0 \times 10^{5}$ | 194 | 26 |
| Morganella morganii | $1.0 \times 10^{8}$ | 1,251 | 267 |
| | $1.0 \times 10^{7}$ | 217 | 32 |
| | $1.0 \times 10^{6}$ | 161 | 7 |
| Mammalian Cells | $1.0 \times 10^{7}$ | 90 | 19 |
| | $1.0 \times 10^{5}$ | 69 | 8 |

A. *Use of B. subtilis ribosomal RNA gene as a probe for Escherichia coli.*

A fragment of the ribosomal RNA genes from *B. subtilis* cloned into pBR313 was used as the probe sequence for hybridization. This ribosomal gene clone is designated p14B1 and is described in Stewart, G. C., Wilson, F. E. and K. F. Bott, "Detailed Physical Mapping of the Ribosomal RNA Genes of *Bacillus subtilis*" [Gene (1982) 19:153-162].

An 18 hour broth- culture (L-Broth which consists of 10g Bacto tryptone, 5g Bacto yeast extract, 0.5 g NaCl and 2 ml of 1M NaOH per liter) of *Escherichia coli* strain MC4100 (Genotype F , ara D139, (lac) U169, re1A, rpsL, thi) Ito, K., Bassford, P. J., Jr. and Beckwith, J., Cell (1981) 24:707-717. The culture was plated on L-agar plates to determine viable count to estimate the number of cells present per sample volume. The culture was also diluted serially to $10^{-6}$. A 100 microliter sample of these dilutions was added to 50 microliters of lysis buffer. (Gegenheimer, P., Watson, N. and D. Apirion. Journal Biological Chemistry, 252:3064-3073) and the sample tube immersed in boiling water bath for two minutes. A five microliter volume of each sample was spotted onto a nitrocellulose membrane (Schleicher & Scheull B85) that had been presoaked in 20XSSC. The filter was dried in a vacuum oven at 80° C for two hours.

The *E. coli* strain which contains plasmid p14B1 was cultured using standard methods and the plasmid DNA isolated. (Maniatis, T., Fritsch, E. F., J. Sambrook. "Molecular Cloning - A Laboratory Manual". CSH - Cold Spring Harbor, New York, pp. 88-93).

The p14B1 DNA was labeled with $^{32}P$ by nick translation as described below. A reaction mixture was prepared consisting of 1.0 $\mu$g of p14B1 DNA, 20 micromoles each of deoxycytidine triphosphate (dCTP), deoxyguanosine triphosphate (dGTP) and deoxythymidine triphosphate (dTTP), 0.05 M Tris-HCl, pH 7.8, 0.005 M magnesium chloride, 0.01 M 2-mercaptoethanol, 0.50 $\mu$g of nuclease free bovine serum albumin (BSA), 2 units of DNA polymerase I (available from BRL, Gaithersburg, MD) 200 picograms DNase I, 0.005$^-$ M magnesium acetate, 250 $\mu$g glycerol, and 4,88 $\mu$moles of $[\alpha^{32}P]$ deoxyadenosine triphosphate (dATP) with a specific activity of 410 Ci/mmol. The total reaction volume was adjusted to 50 microliters with distilled water and incubated at 15°C for one hour. The reaction was stopped by the addition of 5 microliters of 0.3 M Na$_2$EDTA (pH 8.0) and extracted with an equal volume of Tris-saturated phenol. The extracted mixture was chromatographed on a Sephadex® G-50 column (Pharmacia Fine Chemicals, Piscataway, N.J.) to remove the unreacted deosytriphosphates. The $^{32}P$-labeled DNA fragments were contained in a total volume of 400 microliters with a specific activity of 8X10$^7$ cpm per microgram of DNA.

The nitrocellulose membrane described above in this example was pretreated before hybridization by placing the membrane in the heat sealable bag commercially available from Sears Roebuck & Co. and adding 10 ml of a solution consisting of 5XBFP, 5XSSPE, 0.3% SDS and 100 $\mu$g/ml salmon sperm DNA. The membrane was incubated in solution for one hour at 50%C and the prehybridization solution removed. Ten milliliters of a solution containing 5XSSPE. 1XBFP, 100 $\mu$g/ml salmon sperm DNA and 0.3% SDS was prepared. Half of this solution (5 ml) was added to the bag containing the membrane and mixed. The labeled probe DNA was denatured by heating in a solution of 10 mM Tris (pH 7.5), 1 mM EDTA for 10 min. at 95°C and cooling it quickly in an ice bath. The denatured probe DNA was added to the remaining 5 ml of solution at a quantity sufficient to provide 5X10$^6$ cpm per membrane. The solution containing the probe was added to the bag and the bag was heat sealed.

The hybridization mixture was incubated for 16 hours at 50°C. The nitrocellulose membrane was removed from the hybridization solution and washed in a solution of 5XSSPE and 0.5% SDS. This wash was repeated four times at 50°C. The membranes were air dried; subsequently, the membranes were cut up in such a manner as to cut out the filter area encompassed by the spotted sample as well as an equivalent border area. These filter squares were placed into individual scintillation vials containing 5 ml of Aquasol® fluor. Duplicate samples of each cell type and its serial dilutions were counted on a Beckman scintillation counter. The results are shown in Table C.

## Table C

| Number of E. coli Cells in Lysis Buffer Spotted on Membrane | $^{32}$P-CPM-Counts Per Minutes - Measure of Extent of Hybridization of B. subtilis rRNA Gene Fragment to E. coli |
|---|---|
| $2.5 \times 10^7$ Cells | 13,358 |
| $2.5 \times 10^6$ Cells | 1,649 |
| $2.5 \times 10^5$ Cells | 250 |
| $2.5 \times 10^4$ Cells | 94 |
| Blank - No Cells | 65 |

In this experiment, the B. subtilis ribosomal gene fragment probe was able to detect the presence of $2.5 \times 10^5$ Escherichia coli cells by hybridization.

The present invention has been particularly described and exemplified above. Obviously, many other variations and modifications of the invention may be made without departing from the spirit and scope hereof.

## Claims

1. A method for detecting bacteria in a test sample, characterized in the steps of:

    (a) subjecting said test sample to conditions to release RNA from bacteria in the sample,

    (b) contacting the resulting released RNA with a DNA probe having a base sequence which is hybridizable with an RNA base sequence in the nucleic acid content of substantially all of the respective bacteria suspected to be present in said test sample, such contact being accomplished under conditions favorable to hybridization between said DNA probe and said respective bacterial RNA sequences, and

    (c) determining the presence of hybridized probe by adding an antibody, or a fragment thereof, specific for DNA'RNA hybrids and determining the binding of such antibody or fragment to hybrids of said DNA probe and said bacterial RNA sequences.

2. The method of claim 1 wherein said RNA sequence is a ribosomal RNA sequence, such as 16S or 23S RNA.

3. The method of claim 1 or 2 wherein said DNA probe is immobilized.

4. The method of any of claims 1-3 wherein said antibody or fragment to DNA'RNA is labeled with a detectable chemical group, such as an enzyme.

5. The method of any of claims 1-4 wherein said test sample is a mammalian body fluid, such as human urine.

6. A test kit for use in the detection of bacteria in a test sample by nucleic acid hybridization, characterized by:

(1) a DNA probe having a base sequence which is hybridizable with an RNA base sequence in the nucleic acid content of substantially all of the respective bacteria suspected to be present in said test sample, and

(2) an antibody, or a fragment thereof, specific for binding DNA'RNA hybrids.

7. The test kit of claim 6 wherein said RNA sequence is a ribosomal RNA sequence, such as 16S or 23S RNA.

8. The test kit of claim 6 or 7 wherein said DNA probe is immobilized.

9. The test kit of any of claims 6-8 wherein said antibody or fragment to DNA'RNA is labeled with a detectable chemical group, such as an enzyme.

10. Use of the test kit of any of claims 6-9 for the detection of bacteria in a sample of mammalian body fluid, such as a sample of human urine.

**Revendications**

1. Méthode de détection de bactéries dans un échantillon d'essai, caractérisée en ce qu'elle comprend les étapes consistant :

(a) à soumettre ledit échantillon d'essai à des conditions provoquant la libération de l'ARN des bactéries présentes dans l' échantillon,

(b) à mettre en contact l'ARN libéré résultant avec une sonde d'ADN ayant une séquence de bases qui est hybridable avec une séquence de bases d'ARN présente dans l'acide nucléique de pratiquement toutes les bactéries respectives suspectées d'être présentes dans ledit échantillon d'essai, ce contact étant réalisé dans des conditions favorables à l'hybridation entre ladite sonde d'ADN et lesdites séquences d'ARN bactérien respectives, et

(c) à déterminer la présence de la sonde hybridée par addition d'un anticorps, ou d'un de ses fragments, spécifique des hybrides ADN-ARN, et à déterminer la liaison de cet anticorps ou de ce fragment aux hybrides de ladite sonde d'ADN et desdites séquences d'ADN bactérien.

2. Méthode suivant la revendication 1, dans laquelle la séquence d'ARN est une séquence d'ARN ribosomal, telle que l'ARN 16S ou 23S.

3. Méthode suivant la revendication 1 ou 2, dans laquelle la sonde d'ADN est immobilisée.

4. Méthode suivant l'une quelconque des revendications 1 à 3, dans laquelle l'anticorps ou le fragment contre l'ADN-ARN est marqué avec un groupe chimique détectable, tel qu'un enzyme.

5. Méthode suivant l'une quelconque des revendications 1 à 4, dans laquelle l'échantillon d'essai est un liquide biologique de mammifère, tel que l'urine humaine.

6. Kit analytique destiné à être utilisé dans la détection de bactéries dans un échantillon d'essai, par hybridation d'acides nucléiques, caractérisé en ce qu'il comprend :

(1) une sonde d'ADN ayant une séquence de bases qui est hybridable avec une séquence de bases d'ARN présente dans l'acide nucléique de pratiquement la totalité des bactéries respectives suspectées d'être présentes dans ledit échantillon d'essai, et

(2) un anticorps, ou un de ses fragments, présentant une spécificité de liaison avec les hybrides ADN-ARN.

7. Kit analytique suivant la revendication 6, dans lequel la séquence d'ARN est une séquence d'ARN ribosomal, telle que l'ARN 16S ou 23S.

8. Kit analytique suivant la revendication 6 ou 7, dans lequel la sonde d'ADN est immobilisée.

9. Kit analytique suivant l'une quelconque des revendications 6 à 8, dans lequel l'anticorps ou le fragment contre l'ADN-ARN est marqué avec un groupe chimique détectable, tel qu'un enzyme.

10. Utilisation du kit analytique suivant l'une quelconque des revendications 6 à 9 pour la détection de bactéries dans un échantillon de liquide biologique de mammifère, tel qu'un échantillon d'urine humaine.

**Patentansprüche**

1. Verfahren zum Nachweis von Bakterien in einer Untersuchungsprobe, **gekennzeichnet** durch die Schritte:
    (a) Freisetzen der RNA in geeigneter Weise aus dem Bakterium in die Probe;
    (b) Zusammenbringen der freigesetzten RNA mit einer DNA-Sonde, die eine mit einer RNA-Basensequenz im Nukleinsäureanteil von im wesentlichen allen in der Untersuchungsprobe vermuteten Bakterien hybridisierbare Sequenz aufweist, wobei das Zusammenbringen in einer für die Hybridisierung zwischen DNA-Sonde und jeweiligen bakteriellen RNA-Sequenzen geeigneten Weise durchgeführt wird; und
    (c) Nachweis der Anwesenheit der hybridisierten Sonde durch Zugabe eines für DNA'RNA-Hybride spezifischen Antikörpers oder eines Fragmentes davon, und Nachweis der Bindung eines solchen Antikörpers oder Fragmentes an die Hybride der DNA-Sonde mit den bakteriellen RNA-Sequenzen.

2. Verfahren nach Anspruch 1, dadurch **gekennzeichnet,** dass die RNA-Sequenz eine ribosomale RNA-Sequenz, wie 16S oder 23S RNA, ist.

3. Verfahren nach Anspruch 1 oder 2, dadurch **gekennzeichnet,** dass die DNA-Sonde immobilisiert ist.

4. Verfahren nach einem der Ansprüche 1 bis 3, dadurch **gekennzeichnet,** dass der Antikörper oder das Fragment gegen DNA'RNA mit einer detektierbaren chemischen Gruppe, z.B. einem Enzym, markiert ist.

5. Verfahren nach einem der Ansprüche 1 bis 4, dadurch **gekennzeichnet,** dass die Untersuchungsprobe eine Körperflüssigkeit von Säugetieren, z.B. menschlicher Urin, ist.

6. Testkit zur Verwendung beim Nachweis von Bakterien in einer Untersuchungsprobe durch Nukleinsäurehybridisierung, **gekennzeichnet,** durch
    (1) eine DNA-Sonde mit einer Basensequenz, die mit einer RNA-Basensequenz im Nukleinsäuregehalt von im wesentlichen allen Bakterien, die in der Untersuchungsprobe vermutet werden, hybridisierbar ist; und
    (2) einen Antikörper oder ein Fragment davon, spezifisch zur Bindung an DNA'RNA-Hybride.

7. Testkit nach Anspruch 6, dadurch **gekennzeichnet,** dass die RNA-Sequenz eine ribosomale RNA-Sequenz, wie z.B. 16S oder 23S RNA, ist.

8. Testkit nach Anspruch 6 oder 7, dadurch **gekennzeichnet,** dass die DNA-Sonde immobilisiert ist.

9. Testkit nach einem der Ansprüche 6 bis 8, dadurch **gekennzeichnet,** dass der Antikörper oder das Fragment gegen DNA'RNA mit einer detektierbaren chemischen Gruppe, wie z.B. einem Enzym, markiert ist.

**10.** Verwendung des Testkits nach einem der Ansprüche 6 bis 9 zum Nachweis von Bakterien in einer Probe einer Körperflüssigkeit von Säugetieren, z.B. einer menschlichen Urinprobe.